# EUROPEAN PATENT SPECIFICATION

(11) **EP 2 268 599 B1**
(45) Date of publication and mention of the grant of the patent: **21.09.2011**
(21) Application number: 09710309.7
(22) Date of filing: 10.02.2009
(51) Int. Cl.: C07C 33/12, C07C 31/135

(54) **Derivatives of campholenic aldehyde**
Derivate von Kampholenaldehyd
Dérivés de l'aldéhyde campholénique

(30) Priority: 12.02.2008 GB 0802556
(43) Date of publication of application: 05.01.2011
(73) Proprietor: Givaudan SA, 1214 Vernier (CH)
(72) Inventor: MARCH, Sébastien, 1217 Meyrin (CH); BAJGROWICZ, Jerzy, A., 8053 Zürich (CH); DERRER, Samuel, 8117 Fällanden (CH); KRAFT, Philip, 8600 Dübendorf (CH); MUELLER, Urs, 8600 Dübendorf (CH)
(74) Representative: Givaudan Patents
(86) International application number: PCT/CH2009/000054
(87) International publication number: WO 2009/100555

(56) References cited:
- WO-A-92/22518
- WO-A-2008/046239
- HANS MEERWEIN: "Über Ringveränderungen bei der Wasserspaltung aus alicyclischen Alkoholen" JUSTUS LIEBIGS ANNALEN DER CHEMIE, vol. 417, 1918, pages 255-277, XP002531576
- RUPE H ET AL: "1,2,2,3-Tetramethylcyclopentane-1-carbino l and its derivatives" HELVETICA CHIMICA ACTA, VERLAG HELVETICA CHIMICA ACTA. BASEL, CH, vol. 3, 1 January 1920 (1920-01-01), pages 272-298, XP009091171 ISSN: 0018-019X

## Description

This invention refers to novel compounds having patchouli-like odour notes. This invention relates furthermore to a method of their production and to fragrance applications containing them.

Compounds having patchouli-like odour notes are described in the literature, for example, the class of sesquiterpenes that occur naturally in essential oils and which can be isolated by water-steam distillation of a plant or parts of a plant. This process is very cost-intensive, and the quality and the odour as well as the flavour characteristics of the isolated compounds may vary with the climate and the origin of the plant. In addition, patchoulol, which makes up around 35-40 weight % of the essential patchouli oil, is structurally too complex to allow a synthetic approach that could compete with the price of the natural material.

Thus, there is an ongoing demand in the fragrance and flavour industry for new compounds imparting, enhancing, or improving patchouli-like odour notes.

We have now found a novel class of compounds having much sought-after patchouli-like odour notes.

Accordingly, the present invention refers in one of its aspects to the use as flavour or fragrance of a compound of formula (I) 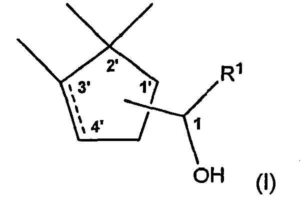 wherein
R¹ is methyl, ethyl or vinyl;
the bond between C-3' and C-4' is a single bond or the dotted line together with the bond between C-3' and C-4' represents a double bond; and
the side chain -CR¹OH is at position 1' or 4' of the ring.

The compounds of formula (I) may comprise several chiral centres and as such may exist as a mixture of stereoisomers, or they may be resolved as isomerically pure forms. Resolving stereoisomers adds to the complexity of manufacture and purification of these compounds and so it is preferred to use the compounds as mixtures of their stereoisomers simply for economic reasons. However, if it is desired to prepare individual stereoisomers, this may be achieved according to methods known in the art, e.g. preparative HPLC and GC, crystallization or by departing from chiral starting materials, e.g. starting from enantiomerically pure or enriched raw materials such as terpenoids, and/or by applying stereoselective synthesis.

Particularly preferred is the use as flavour or fragrance of a compound of formula (I), or a mixture thereof selected from
(1*RS*,1'*S*)-1-(2',2',3'-trimethylcyclopent-3'-en-1'-yl)ethanol,
(1*RS*,1'*S*)-1-(2',2',3'-trimethylcyclopent-3'-enyl)prop-2-en-1-ol,
1-(2',3',3'-trimethylcyclopent-1'-enyl)ethanol,
(+)-(1*RS*,1'*S*,3'*RS*)-1-(2',2',3'-trimethylcyclopentyl)ethanol,
(+)-(1*RS*,1'*S*,3'*RS*)-1-(2',2',3'-trimethylcyclopentyl)prop-2-en-1-ol, and 1-(2,2,3-trimethylcyclopent-3-enyl)propan-1-ol.

The compounds according to the present invention may be used alone or in combination with known fragrances selected from the extensive range of natural and synthetic molecules currently available, such as essential oils and extracts, alcohols, aldehydes and ketones, ethers and acetals, esters and lactones, macrocycles and heterocycles.

In a further embodiment the compounds of formula (I) may be admixed with one or more ingredients or excipients conventionally used in conjunction with fragrances in fragrance aplications, for example, carrier materials, and other auxiliary agents, such as solvents (e.g. dipropyleneglycol (DPG), isopropylmyristate (IPM), triethylcitrate (TEC) and alcohol (e.g. ethanol)), commonly used in the art.

The following list comprises examples of known fragrances, which may be combined with the compounds of the present invention:
- essential oils and extracts, e.g. oak moss absolute, basil oil, tropical fruit oils, such as bergamot oil and mandarin oil, mastic absolute, myrtle oil, palmarosa oil, patchouli oil, petitgrain oil, wormwood oil, lavender oil, rose oil, jasmin oil, ylang-ylang oil and sandalwood oil.
- alcohols, e.g. cis-3-hexenol, cinnamic alcohol, citronellol, Ebanol^{®} (3-methyl-5-(2,2,3-trimethyl-3-cyclopenten-1-yl)-4-penten-2-ol), eugenol, farnesol, geraniol, menthol, nerol, rhodinol, Super Muguet^{™} (6-ethyl-3-methyl-6-octen-1-ol), linalool, phenylethyl alcohol, Sandalore^{®} (5-(2,2,3-trimethyl-3-cyclopentenyl)-3-methylpentan-2-ol), terpineol or Timberol^{®} (1-(2,2,6-trimethylcyclohexyl)hexan-3-ol).
- aldehydes and ketones, e.g. citral, hydroxycitronellal, Lilial^{®} (3-(4-tert-butylphenyl)-2-methylpropanal), methylnonylacetaldehyde, anisaldehyde, allylionone, verbenone, nootkatone, geranylacetone, α-amylcinnamic aldehyde, Georgywood^{™} (1-(1,2,8,8-tetramethyl-1,2,3,4,5,6,7,8-octahydronaphthalen-2-yl)ethanone), hydroxycitronellal, Iso E Super^{®} (1-(2,3,8,8-tetramethyl-1,2,3,4,5,6,7,8-octahydronaphthalen-2-yl)ethanone), Isoraldeine^{®} (4-(2,6,6-trimethyl-2-cyclohexenyl)-3-methyl-3-buten-2-one), Hedione^{®} (methyl (3-oxo-2-pentylcyclopentyl)acetate), maltol, methyl cedryl ketone, and vanillin.
- ethers and acetals, e.g. Ambrox^{®} (3a,6,6,9a-tetramethyldodecahydronaphtho[2,1-b]furan), geranyl methyl ether, rose oxide or Spirambrene (2,2,3',7,7'-pentamethylspiro(1,3-dioxan-5,2'-norcarane)).
- esters and lactones, e.g. benzyl acetate, cedryl actetate, γ-decalactone, Helvetolide^{®} (2-[1-(3,3-dimethylcyclohexyl)ethoxy]-2-methylpropan-1-ol propanoate), γ-undecalactone, vetivenyl acetate, cinnamyl propionate, citronellyl acetate, decyl acetate, dimethylbenzylcarbinyl acetate, ethyl acetoacetate, ethyl acetylacetate, cis-3-hexenyl isobutyrate, linalyl acetate and geranyl acetate.
- macrocycles, e.g. Ambrettolide, Ethylene brassylate or Exaltolide^{®} (oxacyclohexadecan-2-one).
- heterocycles, e.g. isobutylchinoline.

The compounds of the present invention may be used in a broad range of fragranced applications, e.g. in any field of fine and functional perfumery, such as perfumes, household products, laundry products, body care products and cosmetics.

The compounds of formula (I) can be employed in widely varying amounts, depending upon the specific application and on the nature of the composition or application one intends to fragrance, for example the nature and quantity of co-ingredients, and the particular effect that the perfumer seeks. In general, the proportion is typically from 0.001 to 20 weight percent of the application. In one embodiment, compounds of the present invention may be employed in a fabric softener in an amount of from 0.001 to 0.05 weight percent. In another embodiment, compounds of the present invention may be used in an alcoholic solution in amounts of from 0.1 to 30 weight percent, more preferably between 1 and 20 weight percent. However, these values are given only by way of example, since the experienced perfumer may also achieve effects or may create novel accords with lower or higher concentrations, e.g. up to about 50 weight percent based on the composition.

The compounds of the present invention may be employed into a consumer product base by mixing a compound of formula (I), a mixture thereof or fragrance composition comprising it, with the consumer product base, and/or they may, in an earlier step, be entrapped with an entrapment material such as polymers, capsules, microcapsules and nanocapsules, liposomes, film formers, absorbents such as carbon or zeolites, cyclic oligosaccharides and mixtures thereof, and/or they may be chemically bonded to substrates, which together with the substrate forms a precursor, which are adapted to release the compound of formula (I) upon application of an external stimulus such as light, enzyme, or the like, and then mixed with the consumer product base.

The invention additionally provides a method of manufacturing a fragrance application comprising the incorporation of a compound of formula (I) as fragrance ingredient, either by admixing the compound to the consumer product base or by admixing a composition comprising a compound of formula (I) or a precursor thereof, which may then be mixed to the consumer product base, using conventional techniques and methods. Through the addition of an organoleptically acceptable amount of a compound of formula (I) or a mixture thereof, the organoleptic properties of the consumer product base will be improved, enhanced or modified.

The invention furthermore provides a method for improving, enhancing or modifying a consumer product base through the addition thereto of an olfactory acceptable amount of a compound of formula (I), or a mixture thereof.

The invention also provides a fragrance application comprising:
a) as fragrance a compound of formula (I) or a mixture thereof; and
b) a consumer product base.

As used herein, by "consumer product base" is meant a formulation for use as a consumer product to fulfill specific actions, such as cleaning, softening, and caring or the like. Examples of such products include fine perfumery, e.g. perfume and eau de toilette; fabric care, household products and personal care products such as laundry care detergents, rinse conditioner, personal cleansing products, detergent for dishwasher, surface cleaner; laundry products, e.g. softener, bleach, detergent; body care products, e.g. shampoo, shower gel; air care products and cosmetics, e.g. deodorant, and vanishing crème.

According to our best knowledge most of the compounds falling within the definition of formula (I) are not described in the literature and are thus novel in its own right.

Accordingly, the present invention refers in a further aspect to compounds of formula (I) 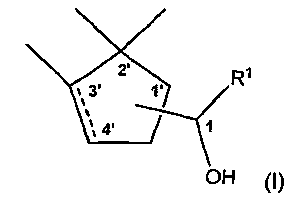 wherein
R¹ is methyl, ethyl or vinyl;
the bond between C-3' and C-4' is a single bond or the dotted line together with the bond between C-3' and C-4' represents a double bond; and
the side chain -CR¹OH is at position 1' or 4' of the ring;
provided that 1-(2,2,3-trimethylcyclopent-3-enyl)ethanol is excluded.
1-(2,2,3-Trimethylcyclopent-3-enyl)ethanol is disclosed in WO2008/046239 as intermediate product for the production of 2,2,3-trimethylcyclopent-3-enecarbaldehyde derivatives.

The compounds of the present invention may be prepared starting from campholytic aldehyde ((*S*)-(+) campholytic aldehyde or (*R*)-(-)-campholytic aldehyde). In case of substitution at position 1', they may be directly prepared by Grignard-reaction with methyl-, ethyl-, or vinylmagnesium halides. The corresponding products with substitution at position 4' are accessible from campholytic aldehyde by acid-mediated rearrangement to afford 2,3,3-trimethylcyclopent-1-enecarbaldehyde and subsequent Grignard-reaction with methyl-, ethyl-, or vinylmagnesium halides. In order to obtain saturated analogues, the C-3'=C-4'-double bond is hydrogenated, for example, under palladium on carbon catalysis, as is well known to the person skilled in the art.

The compositions and methods are now further described with reference to the examples.

The NMR data are given relative to internal SiMe₄ standard.

Example1: (+)-(1*RS*,1'*S*)-1-(2',2',3'-Trimethylcyclopent-3'-en-1'-yl)ethanol (*ca.* 20%*ee*) At room temperature, a 3 M solution of methyl magnesium chloride (66.0 mL, 198 mmol) in THF was added dropwise during 45 min to a stirred solution of (*S*)-campholytic aldehyde [(*S*)-2,2,3-trimethylcyclopent-3-enecarbaldehyde, *ca*. 20%*ee*) in THF (260 mL), upon which the temperature of the reaction mixture rose to 50°C. After refluxing for 3 h, the heating source was removed and the reaction mixture allowed to cool down. Between 0-11°C, by cooling with an ice-water bath, saturated aqueous ammonium chloride solution (55 mL) was added dropwise with stirring, followed by water (200 mL). The product was extracted with ether (2 × 500 mL), and the combined ethereal extracts were washed with water (500 mL) and brine (250 mL). After drying with sodium sulfate and removal of the solvent on a rotary evaporator, the crude product (18.8 g) was purified by flash chromatography (560 g; silica gel, pentane/ether, 4:1, *R*_{f} = 0.40) to provide the (+)-(1*RS*,1'*S*)-1-(2',2',3'-trimethylcyclopent-3'-en-1'-yl)ethanol with ca. 20%ee as a colorless odoriferous liquid.

Odour description: typical clean patchouli scent, woody-camphoraceous-earthy, with slightly spicy accents and fruity-green facets, and some reminiscence to borneol.

IR (neat): ν = 3359 (br. s, νO-H), 1465/1443 (m, δₐₛCH₃), 1373/1359 (s, δₛCH₃), 1143/1126/1010 (s, νC-O).

¹H NMR (CDCl₃): δ = 0.93/0.97/1.04/1.17 (4s, 6 H, 2'-Me₂), 1.19/1.25 (2d, *J* = 6.0 Hz, 3 H, 2-H₃), 1.48 (br. s, 1 H, O-H), 1.59 (br. s, 3 H, 3'-Me), 1.79 (m_{c}, 1 H, 1'-H), 1.81-2.39 (m, 2 H, 5'-H₂), 3.90/3.93 (2quint, *J* = 6.0 Hz, 1 H, 1-H), 5.17/5.27 (2t, *J* = 1.5 Hz, 1 H, 4'-H).

¹³C NMR (CDCl₃): δ = 12.3/12.3 (2q, 3'-Me), 19.6/20.0 (2q, C-2), 23.3/23.8/26.9/27.6 (4q, 2'-Me₂), 31.4/33.2 (2t, C-5'), 46.5/47.0 (2s, C-2'), 56.7/57.5 (2d, C-1'), 68.6/69.8 (2d, C-1), 120.6/121.6 (2d, C-4'), 148.2/149.0 (2s, C-3').

MS (EI): m/z (%) = 41 (9) [C₃H₅⁺], 45 (12) [C₂H₄OH⁺], 55 (8) [C₄H₇⁺], 67 (17) [C₅H₇⁺], 79 (9) [C₆H₇⁺], 91 (6) [C₇H₇⁺], 95 (100) [C₇H₁₁⁺], 105 (3) [C₈H₉⁺], 109 (4) [C₈H₁₃⁺], 121 (37) [M⁺- CH₃- H₂O], 136 (13) [M⁺- H₂O], 139 (1) [M⁺- CH₃], 154 (4) [M⁺].

Polarimetry (c 0.96 in EtOH): [α]D²² = +6.8°, [α]₅₇₈²² = +7.1°, [α]₅₄₆²² = +8.2°, [α]₄₃₆²² = +14.7°, [α]₃₆₅²² = +23.3°.

### Example 2: (+)-(1RS,1'S)-1-(2',2',3'-trimethylcyclopent-3'-enyl)prop-2-en-1-ol

A solution of vinyl bromide (110 g, 1 mol) in 1 L THF is added within 0.5 h into a suspension of magnesium turnings (24.6 g, 1 mol) in 300 mL THF (initiation of the reaction is eased by the addition of ca 0.1 g iodine and heating). The resulting mixture is heated to 60°C for 1 h then cooled to 0 °C. Another solution of campholytic aldehyde (100 g, 0.72 mol, 20.6% ee (*S*)) in 100 mL THF is added drop-wise into the above solution at a rate allowing the mixture temperature to be kept below 20 °C. The resulting mixture is stirred for 1 h at room temperature and then quenched with ice-cold 2M HCl. The aqueous layer is extracted with MTBE and the combined organic layers are dried over MgSO₄, then evaporated yielding the desired alcohol as a yellow liquid (121 g, 99% yield, 93% purity). This material is purified by distillation at 50 °C/0.08 Torr (0.107 mbar) to obtain (+)-(1*RS*,1'*S*)-1-(2',2',3'-trimethylcyclopent-3'-enyl)prop-2-en-1-ol with (ca. 20%ee) as a colorless odoriferous liquid.

Odour description: patchouli, spicy, anisic, badiane, linear; dry down: camphoraceous, woody, patchouli, anisic, black licorice, cinnamic, earthy, honey.

¹H NMR (400 MHz, CDCl₃) δ5.88 (ddd, 17.1 10.3 6.7 Hz, 1H); 5.21 (dt, 17.1 1.4 Hz, 1H); 5.15 (m, 1 H); 5.07 (ddd, 10.2 1.6 1.0 Hz, 1 H); 4.18 (t, 8.3 Hz, 1 H); 2.04 (m, 1 H); 1.91 (m, 1 H); 1.81 (m, 1 H); 1.58 (m, 3H); 1.41 (s, 1H); 1.17 (s, 3H); 0.99 (s, 3H). ¹³C NMR (100 MHz, CDCl₃) δ 148.9 (C^{IV}); 141.3 (CH); 120.7 (CH); 114.6 (CH₂); 75.5 (CH); 55.0 (CH); 47.1 (C^{IV}); 33.0 (CH₂); 27.5 (CH₃); 19.6 (CH₃); 12.3 (CH₃).

MS (EI, m/z) 166 (4, M⁺); 151 (9); 148 (6); 133 (23); 109 (39); 95 (100); 67 (42); 57 (27); 41 (19).

Polarimetry (c 1.06 in EtOH): [α]_{D}²² = +0.4°, [α]₅₇₈²² = +0.5°, [α]₅₄₆²² = +0.6°, [α]₄₃₆²² = +1.3°, [α]₃₆₅ ²² = +2.3°.

### Example 3: 1-(2',3',3'-trimethylcyclopent-1'-enyl)ethanol

A 100 ml two-necked round bottom flask is charged with a solution of campholytic aldehyde (10 g, 0.072 mol) in cyclohexane (30 ml) and Amberlyst 15 (12 g) is added. Under an atmosphere of nitrogen the magnetically stirred mixture is heated to reflux for 18 h. Then, the mixture is allowed to cool, the acid resin is filtered off and the filtrate is reduced *in vacuo* to afford a brown, viscous oil. This crude is purified by bulb-to-bulb distillation (70 °C, 0.08 Torr) to furnish the clean intermediate 2,3,3-trimethylcyclopent-1-enecarbaldehyde. This is converted to the required 1-(2',3',3'-trimethylcyclopent-1'-enyl)ethanol following the procedure outlined in Example 1 and isolated as a colorless odoriferous liquid.

Odour description: minty, agrestic, patchouli, slightly cooling, a bit spicy; dry down: fresh, camphoraceous, patchouli, earthy.

IR (neat): ν = 3339 (br. s, νO-H), 2952/2933 (s, ν CH₂), 1457/1442 (m, δₐₛCH₃), 1377/1360 (s, δₛCH₃), 1061 (s, νC-O).

¹H NMR (CDCl₃): δ = 0.93/0.99 (2s, 6 H, 2'-Me₂), 1.24 (d, *J* = 6.6 Hz, 3 H, 2-H₃), 1.55 (br. s, 3 H, 3'-Me), 1.62 (m_{c}, 2 H, 1'-H₂), ~1.75 (br. s, 1 H, O-H), 2.22-2.51 (m, 2 H, 5'-H₂), 4.66 (q, *J* = 6.0 Hz, 1 H, 1-H).

¹³C NMR (CDCl₃): δ = 9.25 (q, C-2), 21.5 (q, 3'-Me), 25.9/26.2 (2q, 2'-Me₂), 27.0 (t, C-1'), 38.5 (d, C-5'), 47.1 (s, C-2'), 64.8 (d, C-1), 136.2 (s, C-3'), 140.9 (s, C-4'). MS (EI): m/z (%) = 41 (12) [C₃H₅⁺], 43 (100) [C₂H₃O⁺], 45 (6) [C₂H₄OH⁺], 55 (17) [C₄H₇⁺], 67 (11) [C₅H₇⁺], 79 (11) [C₆H₇⁺], 83 (13) [M⁺- CH₂=Me₂-CH₃], 95 (36) [C₇H₁₁⁺], 98 (7)[[M⁺- CH₂=Me₂]]105 (7) [C₈H₉⁺], 109 (6) [C₈H₁₃⁺], 121 (31) [M⁺- CH₃- H₂O], 136 (5)

[M⁺ - H₂O], 139 (48) [M⁺- CH₃], 154 (24) [M⁺].

### Example 4: (+)-(1RS,1'S,3'RS)-1-(2',2',3'-Trimethylcyclopentyl)ethanol (ca. 20%ee)

2,2,3-trimethylcyclopentanecarbaldehyde was prepared directly from α-campholytic aldehyde as follows: To a solution of α-campholytic aldehyde (5 g, 36 mmol, 20.6% ee (S)) in n-butanol/ethyl acetate (1:1, 36 mL), is added palladium on charcoal (5%, 0.3 g). The mixture is magnetically stired for 18 h under an atmosphere of hydrogen (balloon fitted), after which GC-monitoring showed the reaction to be virtually completed. The mixture is filtered through a plug of celite and the filtrate reduced *in vacuo* to afford the required 2,2,3-trimethylcyclopentanecarbaldehyde as colourless odoriferous liquid (5.1 g, quantitative yield, purity >95%). This material was used directly in the procedure described in Example 1 and (+)-(1*RS*,1'*S*)-1-(2',2',3'-Trimethylcyclopentyl)ethanol (ca. 20%ee) was obtained as a colourless, odoriferous liquid (purified by distillation at at 70 °C/0.2 Torr (0.266 mbar)) as a complex mixture of four diastereoisomers.

Odour description: Piney, fruity, agrestic (borneol), woody, patchouli (but not so earthy), a bit spicy, resinous (sprouce).

IR (neat): ν = 3357 (br. s, νO-H), 1468/1455 (m, δₐₛCH₃), 1372/1366 (s, δₛCH₃), 1145/1126/1024 (s, νC-O).

¹H NMR (CDCl₃): δ = 0.64-1.25 (several s and d, 12 H, 1-Me, 2'-Me₂, 3'-Me), 1.05-1.25 (m_{c}, 1 H, 4'-H), 1.41-1.86 (m_{c}, 5 H, 1'-H, 3'-H, 4'-H, 5'-H₂), 3.64-3.97 (4 m_{c}, 1 H, 1-H).

¹³C NMR (CDCl₃): δ = 13.2-16.5 (several q, 2Me), 23.1/23.2/25.0/27.5 (4t, C-4'), 23.6-27.8 (several q, 2Me), 27.8/29.6/31.5/31.6 (4t, C-5'), 41.6/41.8/41.9/42.4 (4s, C-2'), 44.8/44.8/45.7/45.7 (4d, C-4'), 55.0/55.8/57.4/57.8 (4d, C-1'), 68.2/68.7/70.0/70.5 (4d, C-1).

MS (EI): *m*/*z* (%) = 41 (50) [C₃H₅⁺], 45 (42) [C₂H₄OH⁺], 55 (62) [C₄H₇⁺], 69 (100) [C₅H₉⁺], 81 (22) [C₆H₉⁺], 91 (6) [C₇H₇⁺], 95 (38) [C₇H₁₁⁺], 112 (21) [C₈H₁₆⁺], 123 (27) [M⁺- CH₃-H₂O], 138 (11) [M⁺- H₂O], 141 (1) [M⁺- CH₃], 156 (1) [M⁺].

Polarimetry (c 1.02 in EtOH): [α]_{D}²³ = +0.4°, [α]₅₇₈²³ = +0.4°, [a]₅₄₆²³ = +0.5°, [α]₄₃₆²³ = +1.0°, [α]₃₆₅²³ = +1.5°.

### Example 5: (+)-(1RS,1'S,3'RS)-1-(2',2',3'-trimethylcyclopentyl)prop-2-en-1-ol

According to the procedure described in Example 2, (+)-(1*RS*,1'*S*,3'*RS*)-1-(2',2',3'-trimethylcyclopentyl)prop-2-en-1-ol was prepared with vinyl magnesium bromide starting from 2,2,3-trimethylcyclopentanecarbaldehyde and obtained as a colourless, odoriferous liquid (purified by distillation at at 83 °C/0.2 Torr (0.266 mbar)) as a complex mixture of four diastereoisomers.

Odour description: Fruity, metallic, woody, agrestic, patchouli, cooked vegetable.

IR (neat): ν = 3379 (br. s, νO-H). 1642 (w, vC=C), 1468/1450 (m, δₐₛCH₃), 1386/1365 (m, δₛCH₃), 112011109/1066/1045/990/980 (m-s, νC-O).

¹H NMR (400 MHz, CDCl₃) δ = 5.86 (m_{c}, 1 H, 2-H); 5.21 (m_{c}, 1 H, 3-H); 5.08 (m_{c}, 1 H, 3-H); 4.23/4.04/3.97 (3t, 1H, 1-H); 1.87-1.40 (m_{c}, 6 H, 1-OH, 1'-H, 3'-H, 4'-H, 5'-H₂), 1.17-1.07 (m_{c}, 1 H, 4'-H), 1.11-0.64 (several s and d, 9 H, 2'-Me₂, 3'-Me).

¹³C NMR (CDCl₃): δ = 13.3-16.0 (several q, 2Me), 23.4/24.1/25.5/27.7 (4t, C-4'), 24.0-27.7 (several q, 2Me), 29.5/29.9/31.5/31.7 (4t, C-5'), 41.7/41.9/42.1/42.5 (4s, C-2'), 44.6/45.6/45.6/45.7 (4d, C-4'), 53.2/53.7/55.2/55.6 (4d, C-1'), 73.7/74.8/75.0/76.1 (4d, C-1), 114.1/114.3/114.5/114.9 (4t, C-3), 141.5/141.6/141.8/141.9 (4d, C-2).

MS (EI, m/z) 168 (1, M⁺); 153 (1); 135 (3); 111 (56); 95 (26); 69 (100); 55 (52); 41 (33). Polarimetry (c 1.01 in EtOH): [α]_{D}²³ = +0.2°, [α]₅₇₈²³ = +0.2°, [α]₅₄₆²³ = +0.3°, [α]₄₃₆²³ = +0.6°, [α]₃₆₅²³ = +1.0°.

### Example 6: 1-(2,2,3-trimethylcyclopent-3-enyl)propan-1-ol

A solution of α-campholytic aldehyde (10 g, 72 mmol, 20.6% ee (*R*)) in 11 mL THF is added drop-wise into a cooled, mechanically stirred solution of ethylmagnesium bromide (40 mL, 87 mmol, 2.2 M in THF) at a rate allowing the mixture temperature to be kept between -10 °C and 0 °C (ca. 20 min). The resulting solution is stirred for 2h while the temperature is allowed to warm to 0 °C. The resulting heterogeneous mixture is quenched with 100 mL of 2M HCl. The aqueous layer is extracted with MTBE and the combined organic layers are dried over MgSO₄, then evaporated yielding 11.9 g of a yellow liquid (98 % yield). This material can be purified by distillation or chromatography, but was used directly in the next step.

Odour description: Very natural, agrestic, patchouli, a bit earthy/mossy, fruity (fenchyl acetate-type), slightly green.

¹H NMR (400 MHz, CDCl₃) δ 5.26 (broad s, 1H); 3.66 (dt, 8.1, 4.6 Hz, 1H); 2.24 (m, 2H); 1.87 (td, 8.2, 4.8 Hz, 1 H); 1.58 (m, 3H); 1.6-1.4 (m, 2H); 1.42 (broad s, 1 H); 1.01 (s, 3H); 0.96 (t, 7.3 Hz, 3H); 0.95 (s, 3H).

¹³C NMR (100 MHz, CDCl₃) δ 148.2 (C^{IV}); 121.8 (CH); 73.4 (CH); 54.3 (CH); 46.7 (C^{IV}); 30.4 (CH₂); 29.6 (CH₂); 27.0 (CH₃); 20.5 (CH₃); 12.4 (CH₃); 10.3 (CH₃).

MS (EI, m/z) 168 (2, M⁺); 150 (16); 135 (18); 121 (100); 107 (18); 95 (88); 79 (14); 67 (20); 59 (16); 55 (16); 41 (23).

### Example 6: Fougère aromatic green-fruity composition for a shower gel

| Ingredient | parts by weight |
|---|---|
| Allyl amyl glycolate | 6 |
| Ambrofix (dodecahydro-3a,6,6,9a-tetramethyl-naphtho-(2,1-b)-furan) | 2 |
| Amyl salicylate | 60 |
| Carvone laevo ((*R*)-2-methyl-5-(prop-l-en-2-yl)cyclohex-2-enone) | 10 |
| Cedrylacetate | 40 |
| Citronellol | 60 |
| Coumarin | 30 |
| Dihydro eugenol | 6 |
| Dihydro myrcenol (2,6-dimethyloct-7-en-2-ol) | 60 |
| Ethyl vanillin at 10% in dipropyleneglycol (DPG) | 2 |
| Fenchyl acetate | 30 |
| Galaxolide^{®} 50 (1,3,4,6,7,8-hexahydro-4,6,6,7,8,8-hexamethyl-cyclopenta- | |
| gamma-2-benzopyran) at 50% in isopropylmyristate (IPM) | 100 |
| Heliotropine | 10 |
| Hexenol-3-cis | 6 |
| Hexyl acetate | 12 |
| Hexyl cinnamic aldehyde | 100 |
| lonone beta | 40 |
| Iso E super (1-(2,3,8,8-tetramethyl-1,2,3,4,5,6,7,8-octahydronaphthalen-2-yl)- | |
| ethanone) | 60 |
| Labienoxime (2,4,4,7-tetramethyl-6,8-nonadiene-3-one-oxime) | |
| at 1% in IPM-TEC (isopropylmyristate-triethylcitrate mixture 90/10) | 2 |
| Linalool | 160 |
| Maltol isobutyrate at 10% in DPG | 4 |
| Radjanol (2-ethyl-4-(2,2,3-trimethylcyclopent-3-enyl)but-2-en-1-ol) | 16 |
| Stemone^{®} (5-methyl-3-heptanone oxime) | 4 |
| Terpinyl acetate (2-(4-methylcyclohex-3-enyl)propan-2-yl acetate) | 80 |
| (+)-(1*RS*, 1'*S*)-1-(2',2',3'-Trimethylcyclopent-3'-en-1'-yl)ethanol (*ca.* 20%*ee*) | 100 |
| Total: | 1000 |

This fougére fragrance with a spearmint leaf effect and fruity green undertones in the direction of apple, provides a green-aromatic freshness to shower gel formulations, which is greatly enhanced by the patchouli character of (+)-(1*RS*,1'*S*)-1-(2',2',3'-trimethylcyclopent-3'-en-1'-yl)ethanol. Incorporation of this new odorant conveys a natural resinous-woody, balsamic character that enhances the fresh-aromatic theme of the composition without dominating the fragrance with its patchouli note. (+)-(1*RS*,1'*S*)-1-(2',2',3'-Trimethylcyclopent-3'-en-1'-yl)ethanol also stresses the fresh eucalyptus and spearmint effect, but most importantly renders it the construction of a fougére theme possible without incorporation of oak moss. Without the woody-camphoraceous-earthy patchouli scent of (+)-(1*RS*,1'*S*)-1-(2',2',3'-trimethylcyclopent-3'-en-1'-yl)ethanol with its slightly spicy accents and fruity-green facets that ideally form a great part of the aromatic, herbal, green-fruity theme, the fragrance is not recognizable as a fougére anymore, besides that it appears flat and musty.

### Example 7: Fougère aromatic lavender composition for a masculine eau-de-cologne

| Ingredient | parts by weight |
|---|---|
| Agrumex (2-tert-butylcyclohexyl acetate) | 80 |
| Allyl amyl glycolate | 6 |
| Ambrofix (dodecahydro-3a,6,6,9a-tetramethyl-naphtho-(2,1-b)-furan) | 20 |
| Anise oil | 2 |
| Bourgeonal T (3-(4-tert-butylphenyl)propanal) | 6 |
| Clove bud oil | 2 |
| Cyclohexal | 80 |
| Damascenone (1-(2,6,6-trimethylcyclohexa-1,3-dienyl)but-2-en-1-one) | |
| at 10% in dipropyleneglycol (DPG) | 4 |
| Dimethyl phenyl ethyl carbinol (2-methyl-4-phenyl-2-butanol) | 20 |
| Fennaldehyde (3-(4-methoxyphenyl)-2-methylpropanal) | 10 |
| Fixolide (1-(3,5,5,6,8,8-hexamethyl-5,6,7,8-tetrahydronaphthalen-2-yl)- | |
| ethanone) | 120 |
| Floralozone (3-(4-ethylphenyl)-2,2-dimethylpropanal) | 2 |
| Hedione^{®} HC (methyl 2-(3-oxo-2-pentylcyclopentyl)acetate) | 160 |
| Hexenol-3-cis | 4 |
| Irone alpha (4-(2,5,6,6-tetramethylcyclohex-2-enyl)but-3-en-2-one) | 2 |
| Iso E super (1-(2,3,8,8-tetramethyl-1,2,3,4,5,6,7,8-octahydronaphthalen-2-yl)- | |
| ethanone) | 120 |
| Lavender oil | 80 |
| Lemon oil | 40 |
| Liffarome ((Z)-hex-3-enyl methyl carbonate) | 2 |
| Ligustral (2,4-dimethylcyclohex-3-enecarbaldehyde) | 2 |
| Mandarin oil | 14 |
| Methyl cedryl ketone | 80 |
| Radjanol (2-ethyl-4-(2,2,3-trimethylcyclopent-3-enyl)but-2-en-1-ol) | 20 |
| Spearmint oil | 2 |
| Tropional (3-(benzo[d][1,3]dioxol-5-yl)-2-methylpropanal) | 30 |
| Vanillin | 2 |
| (+)-(1*RS*, 1'*S*)-1-(2,2,3-trimethylcyclopent-3-enyl)prop-2-en-1-ol (*ca.* 20%*ee*) | 90 |
| | Total: 1000 |

This composition presents a fresh cologne character with a particular emphasis on a true natural lavender effect. The lavender is blended with a rich woody, ambery and powdery background, while the heart is a soft floral with watery connotations. The addition of (+)-(1*RS*, 1'*S*)-1-(2,2,3-trimethylcyclopent-3-enyl)prop-2-en-1-ol (*ca.* 20%*ee*, Example 2) brings an agrestic patchouli character to the composition. It enhances also the crisp and natural feeling while improving the lift and freshness.

## Claims

1. A compound of formula (I) 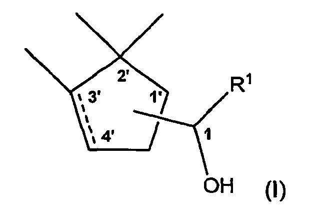 wherein
R¹ is methyl, ethyl or vinyl;
the bond between C-3' and C-4' is a single bond or the dotted line together with the bond between C-3' and C-4' represents a double bond; and
the side chain -CR¹OH is at position 1' or 4' of the ring;
provided that 1-(2,2,3-trimethylcyclopent-3-enyl)ethanol is excluded.

2. A compound according to claim 1 selected from the group consisting of (1*RS*,1'*S*)-1-(2',2',3'-trimethylcyclopent-3'-enyl)prop-2-en-1-ol, 1-(2',3',3'-trimethylcyclopent-1'-enyl)ethanol, (+)-(1*RS*,1'*S*,3'*RS*)-1-(2',2',3'-trimethylcyclopentyl)ethanol, (+)-(1*RS*,1'*S*,3'*RS*)-1-(2',2',3'-trimethylcyclopentyl)prop-2-en-1-ol, and 1-(2,2,3-trimethylcyclopent-3-enyl)propan-1-ol.

3. The use as flavour or fragrance of a compound of formula (I) 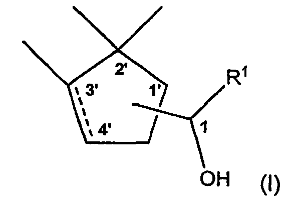 wherein
R¹ is methyl, ethyl or vinyl;
the bond between C-3' and C-4' is a single bond or the dotted line together with the bond between C-3' and C-4' represents a double bond; and
the side chain -CR¹OH is at position 1' or 4' of the ring.

4. The use according to claim 3 wherein the compound of formula (I) is selected from (1*RS*,1'*S*)-1-(2',2', 3'-trimethylcyclopent-3'-en-1'-yl)ethanol, (1*RS*,1'*S*)-1-(2',2',3'-trimethylcyclopent-3'-enyl)prop-2-en-1-ol, 1-(2',3',3'-trimethylcyclopent-1'-enyl)ethanol, (+)-(1*RS*,1'*S*,3'*RS*)-1-(2',2',3'-trimethylcyclopentyl)ethanol, (+)-(1*RS*,1'*S*,3'*RS*)-1-(2',2',3'-trimethylcyclopentyl)prop-2-en-1-ol, and 1-(2,2,3-trimethylcyclopent-3-enyl)propan-1-ol; or a mixture thereof.

5. A method of improving, enhancing or modifying a fragrance application through addition thereto of an olfactory acceptable amount of a compound of formula (I) as defined in claim 3, or a mixture thereof.

6. A fragrance application comprising as odorant a compound of formula (I) as defined in claim 3, or a mixture thereof; and a consumer product base.

7. A fragrance application according to claim 6 wherein the consumer product base is selected from fine fragrances, household products, laundry products, body care products, cosmetics and air care products.

## Patentansprüche

1. Verbindung der Formel (I) 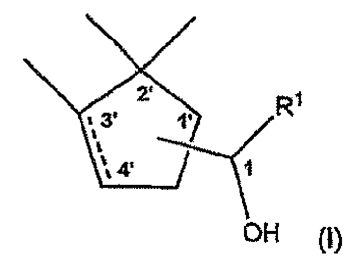 worin
R¹ für Methyl, Ethyl oder Vinyl steht;
die Bindung zwischen C-3' und C-4' eine Einfachbindung ist oder die gestrichelte Linie zusammen mit der Bindung zwischen C-3' und C-4' für eine Doppelbindung steht und
die Seitenkette -CR¹OH in Position 1' oder 4' des Rings steht;
mit der Maßgabe, daß 1-(2,2,3-Trimethylcyclopent-3-enyl)ethanol ausgeschlossen ist.

2. Verbindung nach Anspruch 1, ausgewählt aus der Gruppe bestehend aus (1*RS*,1'*S*)-1-(2',2',3'-Trimethylcyclopent-3'-enyl)prop-2-en-1-ol, 1-(2',3',3'-Trimethylcyclopent-1'-enyl)ethanol, (+)-(1*RS*,1'*S*,3'*RS*)-1-(2',2',3'-Trimethylcyclopentyl)-ethanol, (+)-(1*RS*,1'*S*,3'*RS*)-1-(2',2',3'-Trimethyl-cyclopentyl)prop-2-en-1-ol und 1-(2,2,3-Trimethyl-cyclopent-3-enyl)propan-1-ol.

3. Verwendung einer Verbindung der Formel (I) 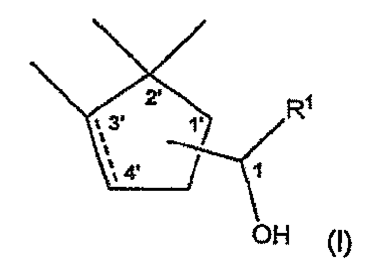 worin
R¹ für Methyl, Ethyl oder Vinyl steht;
die Bindung zwischen C-3' und C-4' eine Einfachbindung ist oder die gestrichelte Linie zusammen mit der Bindung zwischen C-3' und C-4' für eine Doppelbindung steht und
die Seitenkette -CR¹OH in Position 1' oder 4' des Rings steht;
als Geschmacksstoff oder Dufstoff.

4. Verwendung nach Anspruch 3, wobei die Verbindung der Formel (I) unter
(1*RS*,1'*S*)-1-(2',2',3'-Trimethylcyclopent-3'-en-1'-yl)ethanol, (1*RS*,1'*S*)-1-(2',2',3'-Trimethylcyclopent-3'-enyl)prop-2-en-1-ol, 1-(2',3',3'-Trimethylcyclopent-1'-enyl)ethanol, (+)-(1*RS*,1'*S*,3'*RS*)-1-(2',2',3'-Trimethylcyclo-pentyl)ethanol, (+)-(1*RS*,1'*S*,3'*RS*)-1-(2',2',3'-Trimethylcyclo-pentyl)prop-2-en-1-ol und
1-(2,2,3-Trimethylcyclopent-3-enyl)propan-1-ol oder einer Mischung davon ausgewählt ist.

5. Verfahren zur Verbesserung, Verstärkung oder Modifizierung einer Duftstoffanwendung durch Zugabe einer olfaktorische annehmbaren Menge einer Verbindung der Formel (I) gemäß Anspruch 3 oder einer Mischung davon.

6. Duftstoffanwendung, umfassend eine Verbindung der Formel (I) gemäß Anspruch 3 oder eine Mischung davon als Odorans und eine Konsumproduktbasis.

7. Duftstoffanwendung nach Anspruch 6, wobei die Konsumproduktbasis unter Feinduftstoffen, Haushaltsprodukten, Waschmittelprodukten, Körperpflegeprodukten, Kosmetika und Luftbehandlungsprodukten ausgewählt ist.

## Revendications

1. Composé de formule (I) 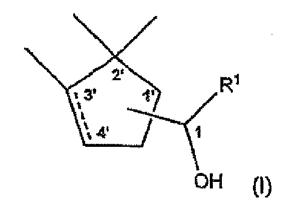 dans laquelle
R¹ est méthyle, éthyle ou vinyle ;
la liaison entre C-3' et C-4' est une simple liaison ou la ligne en pointillés conjointement avec la liaison entre C-3' et C-4' représente une double liaison ; et
la chaîne latérale -CR¹OH est en position 1' ou 4' du cycle ;
à condition que le 1-(2,2,3-triméthylcyclopent-3-ényl)éthanol soit exclu.

2. Composé selon la revendication 1, choisi dans le groupe constitué de (1*RS*,1'*S*)-1-(2',2',3'-triméthylcyclopent-3-ényl)prop-2-én-1-ol, 1-(2',3',3'-triméthylcyclopent-1-ényl)éthanol, (+)-(1*RS*,1'*S*,3'*RS*)-1-(2',2',3'-triméthylcyclopentyl)éthanol, (+)-(1*RS*,1'*S*,3'*RS*)-1-(2',2',3'-triméthylcyclopentyl)prop-2-én-1-ol, et 1-(2,2,3-triméthylcyclopent-3-ényl)propan-1-ol.

3. Utilisation, en tant qu'arôme ou parfum, d'un
composé de formule (I) 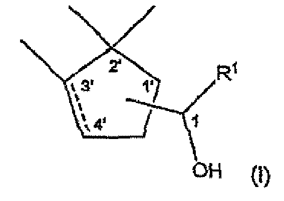 dans laquelle
R¹ est méthyle, éthyle ou vinyle ;
la liaison entre C-3' et C-4' est une simple liaison ou la ligne en pointillés conjointement avec la liaison entre C-3' et C-4' représente une double liaison ; et
la chaîne latérale -CR¹OH est en position 1' ou 4' du cycle.

4. Utilisation selon la revendication 3, **caractérisé en ce que** le composé de formule (I) est choisi parmi
le (1*RS*,1'*S*)-1-(2',2',3'-triméthylcyclopent-3'-én-1'-yl)éthanol, (1*RS*,1'*S*)-1-(2',2',3'-triméthylcyclopent-3'-ényl)prop-2-én-1-ol, 1-(2',3',3'-triméthylcyclopent-1'-ényl)éthanol, (+)-(1*RS*,1'*S*,3'*RS*)-1-(2',2',3'-triméthylcyclopentyl)éthanol, (+)-(1*RS*,1'*S*,3'*RS*)-1-(2',2',3'-triméthylcyclopentyl)prop-2-én-1-ol, et 1-(2,2,3-triméthylcyclopent-3-ényl)propan-1-ol ; ou un mélange de ceux-ci.

5. Méthode pour améliorer, augmenter ou modifier une application de parfum par l'addition à celui-ci d'une quantité acceptable du point de vue olfactif d'un composé de formule (I) tel que défini dans la revendication 3, ou d'un mélange de celui-ci.

6. Application de parfum comprenant, en tant qu'odorisant, un composé de formule (I) tel que défini dans la revendication 3, ou un mélange de celui-ci ; et une base de produit de consommation.

7. Application de parfum selon la revendication 6, **caractérisé en ce que** la base de produit de consommation est choisie parmi les parfums fins, les produits domestiques, les lessives, les produits d'hygiène corporelle, les produits cosmétiques et les produits d'ambiance.
